(19) Europäisches Patentamt
European Patent Office
Office européen des brevets

(11) EP 1 457 487 A1

(12) **EUROPEAN PATENT APPLICATION**
published in accordance with Art. 158(3) EPC

(43) Date of publication:
**15.09.2004 Bulletin 2004/38**

(21) Application number: **02786185.5**

(22) Date of filing: **20.12.2002**

(51) Int Cl.7: **C07D 211/56**, C07M 7/00

(86) International application number:
**PCT/JP2002/013391**

(87) International publication number:
**WO 2003/053929 (03.07.2003 Gazette 2003/27)**

(84) Designated Contracting States:
**AT BE BG CH CY CZ DE DK EE ES FI FR GB GR IE IT LI LU MC NL PT SE SI SK TR**

(30) Priority: **21.12.2001 JP 2001388633**

(71) Applicant: **Toray Fine Chemicals Co., Ltd.**
**Urayasu-shi, Chiba 279-8555 (JP)**

(72) Inventors:
- **FUJINO, Toshihiro**
**Kuwana-shi, Mie 511-0932 (JP)**
- **MORII, Seiji**
**Nagoya-shi, Aichi 467-0043 (JP)**
- **SATO, Haruyo**
**Nagoya-shi, Aichi 454-0926 (JP)**

(74) Representative: **Coleiro, Raymond et al**
**Mewburn Ellis LLP**
**York House**
**23 Kingsway**
**London WC2B 6HP (GB)**

(54) **PROCESS FOR PRODUCTION OF OPTICALLY ACTIVE CIS-PIPERIDINE DERIVATIVES**

(57) An optical-active cis-piperidine derivative of high chemical purity and high optical purity is efficiently produced through optical resolution of a cis-piperidine derivative mixture, racemic cis-piperidine derivative with an optical-active tartaric acid derivative or an optical-active amino acid derivative. For the optical-active tartaric acid derivative, preferred are optical-active di(para-toluoyl)tartaric acid and optical-active di(4-methoxybenzoyl)tartaric acid; and for the optical-active amino acid derivative, preferred is optical-active N-benzenesulfonylphenylalanine.

EP 1 457 487 A1

## Description

### TECHNICAL FIELD

The present invention relates to a method for producing optical-active cis-piperidine derivatives that are useful for medicines or their intermediates.

### BACKGROUND ART

For producing optical-active cis-3-amino-2-phenylpiperidine, known is a method of optical resolution with optical-active mandelic acid (JP-T 8-507297 - the term "JP-T" as used herein means a published Japanese translation of a PCT patent application). The method is an excellent resolution method, but the concentration of the resolution solution is low and the production efficiency is not good. For obtaining products of higher chemical purity and higher optical purity therein, the method requires at least two steps of recrystallization and purification, and the method is problematic in point of its industrial use.

Therefore desired is an efficient industrial method of producing optical-active cis-piperidine derivatives of high chemical purity and high optical purity.

### DISCLOSURE OF THE INVENTION

We, the present inventors have assiduously studied so as to solve the problem and, as a result, have completed the present invention. Specifically, we have found an industrial method of efficiently producing optical-active cis-piperidine derivatives of high chemical purity and high optical purity through optical resolution of racemic cis-piperidine derivatives with an optical-active tartaric acid derivative or an optical-active amino acid derivative.

The invention is a method for producing optical-active cis-piperidine derivatives through optical resolution of a cis-piperidine derivative mixture, racemic cis-piperidine derivative of the following general formulae (1) and (2) with an optical-active tartaric acid derivative or an optical-active amino acid derivative:

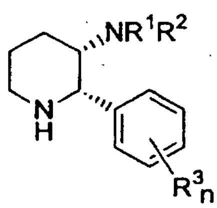 (1) 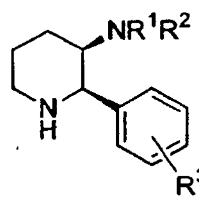 (2)

wherein $R^1$ and $R^2$ each represent a hydrogen atom, or an alkyl group having from 1 to 6 carbon atoms; $R^3$ represents a hydrogen atom, an alkyl group having from 1 to 6 carbon atoms, an alkoxy group having from 1 to 6 carbon atoms, or a halogen atom; n indicates an integer of from 0 to 3.

### BEST MODES OF CARRYING OUT THE INVENTION

The racemic cis-piperidine derivative that is used as the starting material in the invention is a mixture of the compound of formula (1) and the compound of formula (2). The piperidine derivative of formula (1) or (2) includes the following four different optical isomers:

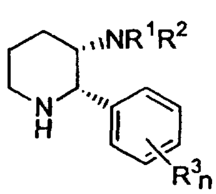 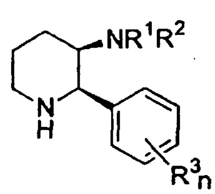 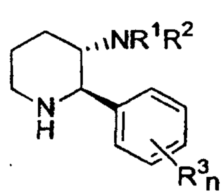 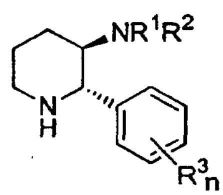

(2S,3S) form (2R,3R) form (2R,3S) form (2S,3R) form

The racemic cis-piperidine derivative in the invention is meant to indicate a mixture of the (2S,3S) form of formula (1) and the (2R,3R) form of formula (2) where the amino group and the phenyl group are in cis-conformation, and the content of any one of the two isomers in the mixture is larger than that of the other by at most 20 %, or that is, the optical purity of the mixture is at most 20 % d.e. The optical-active cis-piperidine derivative is meant to indicate a mixture of the two isomers in which the content of any one of the two is larger than that of the other by at least 80 %, or that is, the optical purity of the mixture is at least 80 % d.e. Specific examples of the cis-piperidine derivative are cis-3-amino-2-phenylpiperidine, cis-3-methylamino-2-phenylpiperidine, cis-3-ethylamino-2-phenylpiperidine, cis-3-amino-2-(4-methylphenyl)piperidine, cis-3-amino-2-(4-chlorophenyl)piperidine, 3-dimethylamino-2-phenylpiperidine, cis-3-amino-2-(2,4-dimethylphenyl)piperidine, cis-3-amino-2-(2-methyl-3-ethylphenyl)piperidine, and these are preferred for use in the invention. Especially preferred is cis-3-amino-2-phenylpiperidine. Racemates of these cis-piperidine derivatives are resolved with an optical resolving agent.

The starting cis-piperidine derivatives may be any ones produced in any methods. For example, they may be produced according to the following reaction formula:

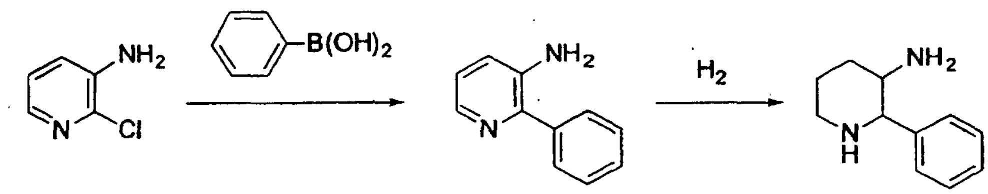


Concretely, 3-amino-2-phenylpyridine obtained by reacting 3-amino-2-chloropyridine with phenylboric acid is hydrogenated in the presence of a hydrogenation catalyst to give racemic cis-3-amino-2-phenylpiperidine. In this process, when the intermediate, 3-amino-2-phenylpyridine is hydrogenated in the presence of a hydrogenation catalyst, then the resulting reaction liquid contains a lot of basic components such as 2-phenylpiperidine, 3-amino-2-cyclohexylpiperidine or 3-amino-2-cyclohexylpyridine. Such basic components behave like cis-3-amino-2-phenylpiperidine. For example, when the reaction liquid is made basic and extracted with an organic solvent and the organic solvent layer is concentrated, then it gives cis-3-amino-2-phenylpiperidine of low chemical purity that contains a lot of basic components such as 2-phenylpiperidine. When the cis-3-amino-2-phenylpiperidine of low chemical purity is used as a starting material and it is optically resolved with an optical-active acidic compound, then the salt of the resulting optical-active cis-3-amino-2-phenylpiperidine and the optical-active acidic compound contains a basic component such as 2-phenylpiperidine. Therefore, unless the salt is further purified, the intended product, optical-active cis-3-amino-2-phenylpiperidine that is obtained by desalting the salt is generally a low-purity product that contains much 2-phenylpiperidine, etc.

The optical resolving agent for use in the invention is meant to include R-form or S-form optical-active tartaric acid derivatives and optical-active amino acid derivatives. Preferably, it is an optical-active compound in which any one optical isomer is excess over the other by at least 95 %, or that is, it has an optical purity of at least 95 % e.e. Using either R-form or S-form depends on the conformation of the intended optical-active cis-piperidine derivative to be produced herein, and it may be determined in accordance with the object of the product.

Preferably, the optical-active tartaric acid derivatives that serve as the optical resolving agent are optical-active tartaric acid amide derivatives of a general formula (3):

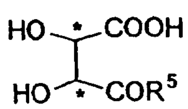
(3)

wherein $R^5$ represents a phenylamino, benzylamino or phenylethylamino group in which the aromatic ring is substituted or unsubstituted; and the carbon atom with * is an asymmetric center;
or optical-active diacyl-tartaric acid derivatives of a general formula (4):

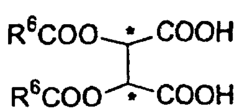
(4)

wherein $R^6$ represents an alkyl group having from 1 to 5 carbon atoms, or a substituted or unsubstituted phenyl or benzyl group; and the carbon atom with * is an asymmetric center.

For example, preferred for use herein are optical-active 4-chlorotartranilic acid, optical-active 4-nitrotartranilic acid, optical-active 2,4-dichlorotartranilic acid, optical-active diacetyltartaric acid, optical-active dibenzoyltartaric acid, optical-active di(paratoluoyl)tartaric acid, optical-active di(metatoluoyl)tartaric acid, optical-active di(orthotoluoyl)tartaric acid, optical-active di(4-methoxybenzoyl)tartaric acid, optical-active di(3-methoxybenzoyl)tartaric acid, optical-active di(2-methoxybenzoyl)tartaric acid. More preferred are optical-active dibenzoyltartaric acid, optical-active di(paratoluoyl)tartaric acid, optical-active di(metatoluoyl)tartaric acid, optical-active di(orthotoluoyl)tartaric acid, optical-active di(4-methoxybenzoyl)tartaric acid, optical-active di(3-methoxybenzoyl)tartaric acid, optical-active di(2-methoxybenzoyl)tartaric acid.

The optical-active amino acid derivatives for use herein are preferably optical-active neutral amino acid derivatives of a general formula (5):

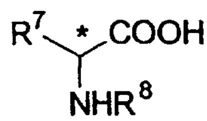

(5)

wherein $R^7$ represents an alkyl group having from 1 to 6 carbon atoms, or a substituted or unsubstituted phenyl, benzyl or phenylethyl group; $R^8$ represents an acyl group having from 1 to 5 carbon atoms, or a benzoyl, benzylcarbonyl, benzenesulfonyl or benzylsulfonyl group in which the aromatic ring is substituted or unsubstituted; and the carbon atom with * is an asymmetric center;

optical-active acidic amino acid derivatives of a general formula (6):

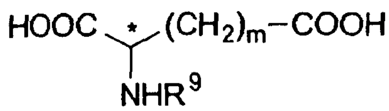

(6)

wherein $R^9$ represents an acyl group having from 1 to 5 carbon atoms, or a benzoyl, benzylcarbonyl, benzenesulfonyl or benzylsulfonyl group in which the aromatic ring is substituted or unsubstituted; m indicates an integer of from 1 to 3; and the carbon atom with * is an asymmetric center;

or optical-active basic amino acid derivatives of a general formula (7):

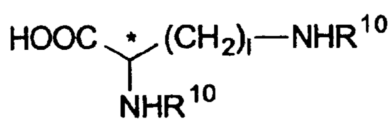

(7)

wherein $R^{10}$ represents an acyl group having from 1 to 5 carbon atoms, or a benzoyl, benzylcarbonyl, benzenesulfonyl or benzylsulfonyl group in which the aromatic ring is substituted or unsubstituted; 1 indicates an integer of from 1 to 5; and the carbon atom with * is an asymmetric center.

More preferred are the optical-active neutral amino acid derivatives of formula (5) wherein $R^7$ is a phenyl or benzyl group, and $R^8$ is a benzoyl, benzylcarbonyl, benzenesulfonyl or benzylsulfonyl group in which the aromatic ring is substituted or unsubstituted; and the optical-active acidic amino acid derivatives of formula (6) wherein $R^9$ is a benzoyl, benzylcarbonyl, benzenesulfonyl or benzylsulfonyl group in which the aromatic ring is substituted or unsubstituted.

For example, even more preferred are optical-active N-formylphenylglycine, optical-active N-acetylphenylglycine, optical-active N-benzoylphenylglycine, optical-active N-benzenesulfonylphenylglycine, optical-active N-toluenesulfonylphenylglycine, optical-active N-benzylsulfonylphenylglycine, optical-active N-formylphenylalanine, optical-active N-acetylphenylalanine, optical-active N-benzoylphenylalanine, optical-active N-benzenesulfonylphenylalanine, optical-active N-toluenesulfonylphenylalanine, optical-active N-benzylsulfonylphenylalanine, optical-active N-formylaspartic acid, optical-active N-acetylaspartic acid, optical-active N-benzoylaspartic acid, optical-active N-benzenesulfonylaspartic acid, optical-active N-toluenesulfonylaspartic acid, optical-active N-benzylsulfonylaspartic acid, optical-active N-formylglutamic acid, optical-active N-acetylglutamic acid, optical-active N-benzoylglutamic acid, optical-active N-benzenesulfonylglutamic acid, optical-active N-toluenesulfonylglutamic acid, optical-active N-benzylsulfonylglutamic acid; and even more preferred are optical-active N-toluenesulfonylphenylglycine, optical-active N-benzenesulfonylphenylglycine, optical-active N-formylphenylalanine, optical-active N-benzoylphenylalanine, optical-active N-benzenesulfonylphenylalanine, optical-active N-toluenesulfonylphenylanaline, optical-active N-benzoylaspartic acid, optical-active N-benzenesulfonylaspartic acid, optical-active N-toluenesulfonylaspartic acid, optical-active N-benzenesulfonylglutamic acid, optical-active N-toluenesulfonylglutamic acid.

In optical resolution of racemic cis-3-amino-2-phenylpiperidine of low chemical purity that contains impurities such as 2-phenylpiperidine, 3-amino-2-cyclohexylpiperidine or 3-amino-2-cyclohexylpyridine, it is desirable to use the optical resolving agent selected from the above-mentioned optical-active tartaric acid derivatives of formula (3) or (4) or optical-active amino acid derivatives of formula (5), (6) or (7), especially optical-active N-benzenesulfonylphenylalanine as it gives optical-active cis-3-amino-2-phenylpiperidine of high chemical purity and high optical purity.

The amount of the optical resolving agent to be used is preferably from 0.8 to 2.5 molar times, more preferably from 0.9 to 2.0 molar times the amount of the racemic cis-piperidine derivative. The optical resolving agent may be combined with an inorganic acid such as hydrochloric acid or sulfuric acid or with optical-inactive acetic acid or propionic acid. In that case, the amount of the optical resolving agent to be used may be reduced.

The solvent for optical resolution (hereinafter referred to as "optical resolution solvent") must not react with substrates. It varies, depending on the type of the starting racemic cis-piperidine derivative and the resolving agent used, for which, for example, preferred are water, alcohols having from 1 to 8 carbon atoms, nitriles such as acetonitrile, ethers such as tetrahydrofuran, esters such as ethyl acetate, isopropyl acetate, and halides such as chloroform. These may be used either singly or as a mixed solvent thereof. More preferred are water, methanol, ethanol, propanol, tetrahydrofuran, acetonitrile, ethyl acetate or their mixtures.

The temperature for optical resolution varies depending on the type of the starting racemic cis-piperidine derivative, the resolving agent and the solvent used, and generally falls between 0°C and the boiling point of the reaction system.

For optically resolving it, the starting racemic cis-piperidine derivative is fed into a reactor along with a resolving agent and a solvent thereinto, and the precipitated salt is taken out through filtration. For it, employable is any of a method of feeding the compounds all at a time into the reactor; a method of first feeding the starting racemic cis-piperidine derivative and a solvent followed by feeding a resolving agent thereinto with stirring; or a method of first feeding a solvent and a resolving agent followed by feeding the starting racemic cis-piperidine derivative thereinto with stirring. Varying depending on the type of the starting racemic cis-piperidine derivative, the resolving agent and the solvent used, the most preferred method for the compounds to be used may be selected. After the compounds have been fed into a reactor, these are dissolved under heat; or after they have reached a state of full equilibrium while in slurry, they are gradually cooled and the precipitated crystal is taken out through filtration to isolate it. In case where the adhesion of the mother liquid thereto has a significant influence on the crystal or where products of especially high optical purity are to be produced, a solvent is again added to the crystal to dissolve it or wash it in slurry, and the precipitated crystal is taken out through filtration. Through the process, the optical purity of the crystal obtained can be readily increased.

Isolating the optical-active cis-piperidine derivative may be effected in any ordinary manner. For example, when a salt of the optical-active cis-piperidine derivative and the resolving agent is made alkaline with an alkali added thereto with stirring in water and toluene, then the optical-active sic-piperidine derivative migrates into the toluene layer. With that, the toluene layer is concentrated and distilled to obtain the optical-active cis-piperidine derivative of high purity. The resolving agent remaining in the basic aqueous solution may be recovered and recycled. The process is advantageous for saving natural resources in that it efficiently gives optical-active cis-piperidine derivatives of high optical purity and that the resolving agent used therein may be recovered and recycled. When the recovered unnecessary optical isomer, cis-piperidine derivative is racemized and recycled therein, then the process is more advantageous for saving natural resources.

EXAMPLES

The invention is described in more detail with reference to the following Examples, to which, however, the invention is not limited.

Analyzing optical-active piperazine derivatives for their optical purity varies depending on the derivative to be analyzed. In the Examples, the derivative is reacted with optical-active tartaric acid derivative anhydride (Toray's product) to convert it into an optical-active tartaric acid derivative thereof, according to the following reaction formula, and the resulting derivative is analyzed through HPLC with ODS columns.

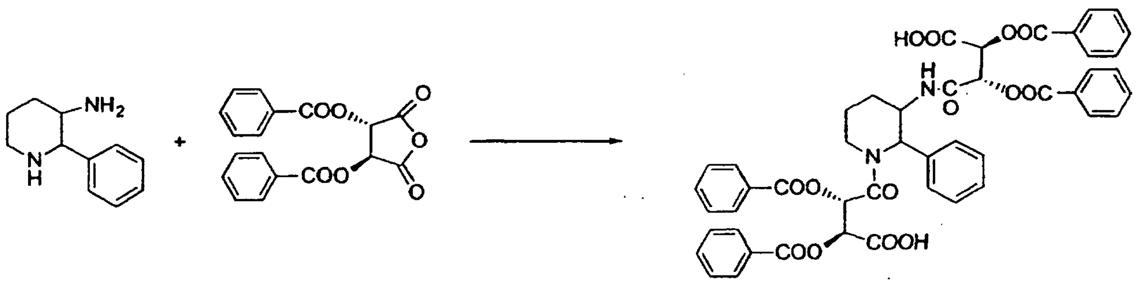

Optical Purity Calculation (for (R,R) > (S,S)):

{[(R,R) peak area - (S,S) peak area]/[(R,R) peak area + (S,S) peak area]} × 100 (% d.e.)

Example 1:

1.05 g (4 mmols) of triphenyl phosphine, 0.224 g (1 mmol) of palladium acetate and 750 ml of toluene were fed into a 3000-ml reactor equipped with a thermometer, a condenser and a stirrer, and stirred at 20°C for 15 minutes. Next, 96.8 g (0.933 mols) of triphenylboroxine, 100.0 g (0.778 mols) of 3-amino-2-chloropyridine, and 950.0 g (1.9 mols) of aqueous 21 % sodium carbonate solution were fed into the reactor. The mixed slurry was heated and stirred at its boiling point for 4 hours. The reaction liquid was cooled to room temperature, and subjected to liquid-liquid separation to remove the aqueous layer. The toluene layer was washed with 200 g of water and then concentrated under reduced pressure to obtain 3-amino-2-phenylpyridine (purity 97.0 %).

20 g of the thus-obtained 3-amino-2-phenylpyridine, 100 ml of 35 % hydrochloric acid, 300 ml of water, and 20 g of 5 % Pt/C (50 % hydrate) were put into a 1000-ml glass autoclave, and stirred in a hydrogen atmosphere at about 30°C under a pressure of 0.3 to 0.4 MPa for 13 hours. After the reaction, this was filtered to remove the catalyst. The filtrate was adjusted to have a pH of 12 with aqueous 48 % sodium hydroxide solution added thereto, and then extracted with 300 ml of chloroform. The aqueous layer was removed through liquid-liquid separation, and the chloroform layer was concentrated to obtain 19.4 g of oily, racemic cis-3-amino-2-phenylpiperidine. Its chemical purity was 82.4 %.

5.71 g (26.7 mmols) of the thus-obtained racemic cis-3-amino-2-phenylpiperidine, 8.16 g (26.7 mmols) of N-benzenesulfonyl-L-phenylalanine, and 22.6 g of methanol were fed into a 100-ml flask equipped with a stirrer, a thermometer and a condenser. This was heated at 50 to 55°C, and stirred for 1 hour at the temperature, and then cooled to 20°C over a period of about 2 hours. The precipitated crystal was taken out through filtration, and dried to obtain 5.36 g of a salt. In the salt, the content of 3-amino-2-phenylpiperidine was 33.9%, and the optical purity of the (2R, 3R) isomer was 93 % d. e. Next, 0.5 g of aqueous 48 % sodium hydroxide solution, 1 ml of water and 5 ml of chloroform were added to 0.3 g of the salt, and stirred at room temperature to extract out 3-amino-2-phenylpiperidine. After liquid-liquid separation, the chloroform layer was analyzed through gas chromatography, which confirmed that the purity of 3-amino-2-phenylpiperidine was 99.4 % except the solvent peak.

Example 2:

5.71 g (26.7 mmols) of racemic cis-3-amino-2-phenylpiperidine that had been obtained in Example 1, 8.16 g (26.7 mmols) of N-benzenesulfonyl-D-phenylalanine, and 22.6 g of methanol were fed into a 100-ml flask equipped with a stirrer, a thermometer and a condenser. This was heated at 50 to 55°C, and stirred for 1 hour at the temperature, and then cooled to 20°C over a period of about 2 hours. The precipitated crystal was taken out through filtration, and dried to obtain 5.36 g of a salt. In the salt, the content of 3-amino-2-phenylpiperidinewas 34.1 %, and the optical purity of the (2S,3S) isomer was 92 % d.e. Next, 0.5 g of aqueous 48 % sodium hydroxide solution, 1 ml of water and 5 ml of chloroform were added to 0. 3 g of the salt, and stirred at room temperature to extract out 3-amino-2-phenylpiperidine. After liquid-liquid separation, the chloroform layer was analyzed through gas chromatography, which confirmed that the purity of 3-amino-2-phenylpiperidine was 99.3 % except the solvent peak.

Example 3:

5.71 g (26.7 mmols) of racemic cis-3-amino-2-phenylpiperidine that had been obtained in Example 1, 5.71 g (18.7 mmols) of N-benzenesulfonyl-D-phenylalanine, and 14.1 g of methanol were fed into a 100-ml flask equipped with a stirrer, a thermometer and a condenser. This was heated at 50 to 55°C, and stirred for 1 hour at the temperature, and then cooled to 20°C over a period of about 2 hours. The precipitated crystal was taken out through filtration, and dried to obtain 4.89 g of a salt. In the salt, the content of 3-amino-2-phenylpiperidine was 34.3 %, and the optical purity of the (2S,3S) isomer was 94 % d.e. Next, 0.5 g of aqueous 48 % sodium hydroxide solution, 1 ml of water and 5 ml of chloroform were added to 0.3 g of the salt, and stirred at room temperature to extract out 3-amino-2-phenylpiperidine. After liquid-liquid separation, the chloroform layer was analyzed through gas chromatography, which confirmed that the purity of 3-amino-2-phenylpiperidine was 99.4 % except the solvent peak.

Example 4:

5.80 g (26.7 mmols) of racemic cis-3-amino-2-phenylpiperidine having a purity of 81.1 % that had been obtained in the same manner as in Example 1, 5.71 g (18.7 mmols) of N-benzenesulfonyl-D-phenylalanine, 0.48 g (8.0 mmols) of acetic acid and 14.1 g of methanol were fed into a 100-ml flask equipped with a stirrer, a thermometer and a condenser. This was heated at 50 to 55°C, and stirred for 1 hour at the temperature, and then cooled to 20°C over a period of about 2 hours. The precipitated crystal was taken out through filtration, and dried to obtain 5.51 g of a salt. In the salt, the content of 3-amino-2-phenylpiperidine was 35.7 %, and the optical purity of the (2S,3S) isomer was 93 % d.e. Next, 0.5 g of aqueous 48 % sodium hydroxide solution, 1 ml of water and 5 ml of chloroform were added to 0.3 g of the salt, and stirred at room temperature to extract out 3-amino-2-phenylpiperidine. After liquid-liquid separation, the chloroform layer was analyzed through gas chromatography, which confirmed that the purity of 3-amino-2-phenylpiperidine was 99.2 % except the solvent peak.

Example 5:

5.80 g (26.7 mmols) of racemic cis-3-amino-2-phenylpiperidine having a purity of 81.1 % that had been obtained in the same manner as in Example 1, 8.53 g (26.7 mmols) of N-p-toluenesulfonyl-L-phenylalanine, and 22.6 g of methanol were fed into a 100-ml flask equipped with a stirrer, a thermometer and a condenser. This was heated at 50 to 55°C, and stirred for 1 hour at the temperature, and then cooled to 20°C over a period of about 2 hours. The precipitated crystal was taken out through filtration, and dried to obtain 4.72 g of a salt. In the salt, the content of 3-amino-2-phenylpiperidine was 31.4 %, and the optical purity of the (2S,3S) isomer was 80 % d.e. Next, 0.5 g of aqueous 48 % sodium hydroxide solution, 1 ml of water and 5 ml of chloroform were added to 0.3 g of the salt, and stirred at room temperature to extract out 3-amino-2-phenylpiperidine. After liquid-liquid separation, the chloroform layer was analyzed through gas chromatography, which confirmed that the purity of 3-amino-2-phenylpiperidine was 92.3 % except the solvent peak.

Example 6:

5.80 g (26.7 mmols) of racemic cis-3-amino-2-phenylpiperidine having a purity of 81.1 % that had been obtained in the same manner as in Example 1, 10.31 g (26.7 mmols) of di(paratoluoyl)-L-tartaric acid, and 48.0 g of methanol were fed into a 100-ml flask equipped with a stirrer, a thermometer and a condenser. This was heated at 50 to 55°C, and stirred for 1 hour at the temperature, and then cooled to 20°C over a period of about 2 hours. The precipitated crystal was taken out through filtration, and dried to obtain 7.46 g of a salt. In the salt, the content of 3-amino-2-phenylpiperidine was 28.7 %, and the optical purity of the (2S,3S) isomer was 96 % d.e. Next, 0.5 g of aqueous 48 % sodium hydroxide solution, 1 ml of water and 5 ml of chloroform were added to 0.3 g of the salt, and stirred at room temperature to extract out 3-amino-2-phenylpiperidine. After liquid-liquid separation, the chloroform layer was analyzed through gas chromatography, which confirmed that the purity of 3-amino-2-phenylpiperidine was 94.7 % except the solvent peak.

Example 7:

10 ml of methanol, 1.8 g (10 mmols) of racemic cis-3-amino-2-phenylpiperidine, and 4.2 g (10 mmols) of di (4-methoxybenzoyl)-L-tartaric acid (Toray's product) were fed into a 50-ml three-neck flask equipped with a stirrer, a condenser and a thermometer, and dissolved with stirring at 60°C. Next, this was cooled to room temperature with stirring for about 1 hour, and then further stirred for 2 hours. The precipitated crystal was taken out through filtration and dried to obtain 2.7 g of a salt. The yield of the salt was 45 %, and the optical purity of the (2S,3S) isomer was 50.4

% d.e. The precipitated crystal was recrystallized in 20 ml of methanol, and the resulting crystal was taken out through filtration and dried to obtain 1.8 g of a salt. The yield of the salt was 65 %: and the optical purity of the (2S,3S) isomer of cis-3-amino-2-phenylpiperidine in the salt was 98.6 % d.e.

Examples 8 to 11:

7 ml of methanol, 5 mmols of the following optical-active L-aspartic acid derivative, and 0.9 g (5 mmols) of racemic cis-3-amino-2-phenylpiperidine were fed into a 20-ml sample bottle with a stopper and a stirrer, and heated at 50°C for 10 minutes, then cooled to room temperature, and further stirred for 2 hours. The precipitated crystal was taken out through filtration and dried, and the optical purity of cis-3-amino-2-phenylpiperidine in the salt was determined. The result is given in Table 1.
Optical-active L-aspartic acid derivative:

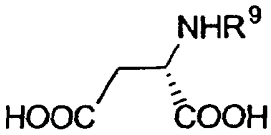

Table 1

| Example | $R^9$ | Yield of Precipitated Salt | Optical Purity of Precipitated Crystal |
|---|---|---|---|
| 8 | acetyl | 52 % | (2R,3R) 69.4 % d.e. |
| 9 | paratoluoyl | 24 % | (2R,3R) 71.3 % d.e. |
| 10 | paratoluenesulfonyl | 45 % | (2S,3S) 52.8 % d.e. |
| 11 | benzylsulfonyl | 56 % | (2R,3R) 59.1 % d.e. |

The precipitated crystal was recrystallized in methanol. Thus obtained, the optical purity of optical-active cis-3-amino-2-phenylpiperidine in the salt was 98 % d.e.

Example 12:

10 ml of methanol, 1.8 g (10 mmols) of racemic cis-3-amino-2-phenylpiperidine, and 3.9 g (10 mmols) of di (paratoluoyl)-L-tartaric acid were fed into a 50-ml three-neck flask equipped with a stirrer, a condenser and a thermometer, and dissolved with stirring at 60°C. Next, this was cooled to room temperature with stirring for about 1 hour, and then further stirred for 2 hours. The precipitated crystal was taken out through filtration and dried to obtain 2.5 g of a salt. The yield of the salt was 45 %, and the optical purity of the (2S, 3S) isomer of cis-3-amino-2-phenylpiperidine in the salt was 88.7 % d.e. The precipitated crystal was recrystallized in 10 ml of methanol, and the resulting crystal was taken out through filtration and dried to obtain 2.3 g of a salt. The yield of the salt was 91 %; and the optical purity of the (2S,3S) isomer of cis-3-amino-2-phenylpiperidine in the salt was 99.3 % d.e.

2.3 g of the precipitated crystal and 20 ml of toluene were fed into a 50-ml separating funnel, and 10 ml of aqueous 5 % sodium hydroxide solution was added to it and well shaken. The toluene layer was separated, and the aqueous layer was further extracted with 20 ml of toluene. The two toluene layers were combined and concentrated with an evaporator to obtain 0.7 g of a concentrate. The chemical purity of (2S,3S)-3-amino-2-phenylpiperidine in the concentrate was 98.1 % (purity through GC analysis purity with no solvent peak), and the optical purity thereof was 99.3 % d.e.

Example 13:

Not racemic cis-3-amino-2-phenylpiperidine but racemic cis-3-methylamino-2-phenylpiperidine was optically

resolved in the same manner as in Example 7, and the precipitated crystal was taken out through filtration and dried to obtain 2.7 g of a salt. The yield of the salt was 45 %, and the optical purity of the (2S,3S) isomer of cis-3-methylamino-2-phenylpiperidine in the salt was 50.4 % d.e. The precipitated crystal was recrystallized in 20 ml of methanol, and the resulting crystal was taken out through filtration and dried to obtain 1.8 g of a salt. The yield of the salt was 65 %; and the optical purity of the (2S,3S) isomer of cis-3-methylamino-2-phenylpiperidine in the salt was 98.1 % d.e. This was again recrystallized in 10 ml of methanol to obtain a crystal of the salt in which the optical purity of the (2S,3S) isomer of cis-3-methylamino-2-phenylpiperidine was 99.7 % d.e.

Example 14:

7 ml of water, 1.4 g (5 mmols) of optical-active paratoluenesulfonyl-L-aspartic acid, and 0.9 g (5 mols) of racemic cis-3-amino-2-phenylpiperidine were fed into a 20-ml sample bottle with a stopper and a stirrer, and heated at 50°C for 10 minutes, then cooled to room temperature, and further stirred for 2 hours. The precipitated crystal was taken out through filtration and dried to obtain 1.0 g of a salt. The yield of the salt was 40.1%, and the optical purity of the (2S,3S) isomer in the salt was 45.2 % d.e. The precipitated crystal was recrystallized twice in water to obtain a crystal of the salt in which the optical purity of (2S,3S)-3-amino-2-phenylpiperidine was 99.1 % d.e.

Comparative Example 1:

5.80 g (26.7 mmols) of racemic cis-3-amino-2-phenylpiperidine having a purity of 81.1 % that had been obtained in the same manner as in Example 1, 4.06 g (26.7 mmols) of L-mandelic acid, and 148.9 g of acetonitrile were fed into a 100-ml flask equipped with a stirrer, a thermometer and a condenser. This was heated at 50 to 55°C, and stirred for 1 hour at the temperature, and then cooled to 20°C over a period of about 2 hours. The precipitated crystal was taken out through filtration, and dried to obtain 3.21 g of a salt. In the salt, the content of 3-amino-2-phenylpiperidine was 34. 9 %, and the optical purity of the (2S,3S) isomer was 52 % d. e. Next, 0.5 g of aqueous 48 % sodium hydroxide solution, 1 ml of water and 5 ml of chloroform were added to 0.3 g of the salt, and stirred at room temperature to extract out 3-amino-2-phenylpiperidine. After liquid-liquid separation, the chloroform layer was analyzed through gas chromatography, which confirmed that the purity of 3-amino-2-phenylpiperidine was 90.4 % except the solvent peak.

INDUSTRIAL APPLICABILITY

According to the present invention, optical-active cis-piperidine derivatives of high optical purity can be readily produced.

## Claims

1. A method for producing optical-active cis-piperidine derivatives through optical resolution of a cis-piperidine derivative mixture, racemic cis-piperidine derivative of the following general formulae (1) and (2) with an optical-active tartaric acid derivative or an optical-active amino acid derivative:

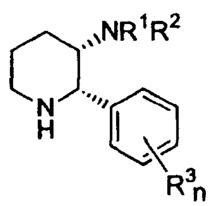 (1) 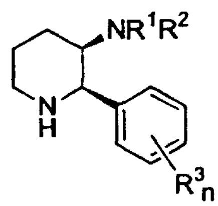 (2)

wherein $R^1$ and $R^2$ each represent a hydrogen atom, or an alkyl group having from 1 to 6 carbon atoms; $R^3$ represents a hydrogen atom, an alkyl group having from 1 to 6 carbon atoms, an alkoxy group having from 1 to 6 carbon atoms, or a halogen atom; n indicates an integer of from 0 to 3.

2. The method for producing optical-active cis-piperidine derivatives as claimed in claim 1, wherein the optical-active tartaric acid derivative is any of optical-active tartaric acid amide derivatives of a general formula (3):

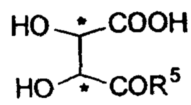

(3)

wherein $R^5$ represents a phenylamino, benzylamino or phenylethylamino group in which the aromatic ring is substituted or unsubstituted; and the carbon atom with * is an asymmetric center;
or optical-active diacyl-tartaric acid derivatives of a general formula (4):

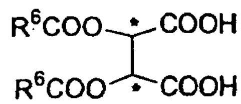

(4)

wherein $R^6$ represents an alkyl group having from 1 to 5 carbon atoms, or a phenyl or benzyl group in which the aromatic ring is substituted or unsubstituted; and the carbon atom with * is an asymmetric center.

**3.** The method for producing optical-active cis-piperidine derivatives as claimed in claim 2, wherein the optical-active tartaric acid derivative is at least one selected from a group consisting of optical-active dibenzoyltartaric acid, optical-active di(paratoluoyl)tartaric acid, optical-active di(metatoluoyl)tartaric acid, optical-active di(orthotoluoyl) tartaric acid, optical-active di(4-methoxybenzoyl)tartaric acid, optical-active di(3-methoxybenzoyl)tartaric acid, and optical-active di(2-methoxybenzoyl)tartaric acid.

**4.** The method for producing optical-active cis-piperidine derivatives as claimed in claim 1, wherein the optical-active amino acid derivative is any of optical-active neutral amino acid derivatives of a general formula (5):

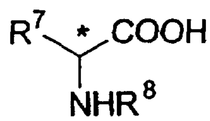

(5)

wherein $R^7$ represents an alkyl group having from 1 to 6 carbon atoms, or a substituted or unsubstituted phenyl, benzyl or phenylethyl group; $R^8$ represents an acyl group having from 1 to 5 carbon atoms, or a benzoyl, benzylcarbonyl, benzenesulfonyl or benzylsulfonyl group in which the aromatic ring is substituted or unsubstituted; and the carbon atom with * is an asymmetric center;
optical-active acidic amino acid derivatives of a general formula (6):

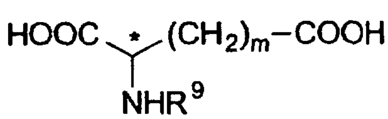

(6)

wherein $R^9$ represents an acyl group having from 1 to 5 carbon atoms, or a benzoyl, benzylcarbonyl, benzenesulfonyl or benzylsulfonyl group in which the aromatic ring is substituted or unsubstituted; m indicates an integer of from 1 to 3; and the carbon atom with * is an asymmetric center;
or optical-active basic amino acid derivatives of a general formula (7):

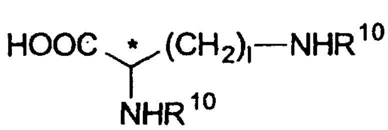

(7)

wherein $R^{10}$ represents an acyl group having from 1 to 5 carbon atoms, or a benzoyl, benzylcarbonyl, benzenesulfonyl or benzylsulfonyl group in which the aromatic ring is substituted or unsubstituted; 1 indicates an integer of from 1 to 5; and the carbon atom with * is an asymmetric center.

**5.** The method for producing optical-active cis-piperidine derivatives as claimed in claim 4, wherein $R^7$ in formula (5) is a phenyl or benzyl group, and $R^8$ is a benzoyl, benzylcarbonyl, benzenesulfonyl or benzylsulfonyl group in which the aromatic ring is substituted or unsubstituted.

**6.** The method for producing optical-active cis-piperidine derivatives as claimed in claim 4, wherein $R^9$ in formula (6) is a benzoyl, benzylcarbonyl, benzenesulfonyl or benzylsulfonyl group in which the aromatic ring is substituted or unsubstituted.

**7.** The method for producing optical-active cis-piperidine derivatives as claimed in claim 5 or 6, wherein the optical-active amino acid derivative is at least one selected from a group consisting of optical-active N-benzoylphenylglycine, optical-active N-benzenesulfonylphenylglycine, optical-active N-toluenesulfonylphenylglycine, optical-active N-benzylsulfonylphenylglycine, optical-active N-benzoylphenylalanine, optical-active N-benzenesulfonylphenylalanine, optical-active N-toluenesulfonylphenylalanine, optical-active N-benzylsulfonylphenylalanine, optical-active N-benzoylaspartic acid, optical-active N-benzenesulfonylaspartic acid, optical-active N-toluenesulfonylaspartic acid, optical-active N-benzylsulfonylaspartic acid, optical-active N-benzoylglutamic acid, optical-active N-benzenesulfonylglutamic acid, optical-active N-toluenesulfonylglutamic acid, optical-active N-benzylsulfonylglutamic acid.

**8.** The method for producing optical-active cis-piperidine derivatives as claimed in claim 5, wherein the optical-active amino acid derivative is optical-active N-benzenesulfonylphenylalanine.

**9.** The method for producing optical-active cis-piperidine derivatives as claimed in any one of claims 1 to 8, wherein the optical resolving agent is water, methanol, ethanol, propanol, tetrahydrofuran, acetonitrile, ethyl acetate or their mixture.

**10.** The method for producing optical-active cis-piperidine derivatives as claimed in any one of claims 1 to 9, wherein the racemic cis-piperidine derivative of formula (1) is racemic cis-3-amino-2-phenylpiperidine obtained through hydrogenation of 3-amino-2-phenylpyridine in the presence of a hydrogenation catalyst, and the optical-active cis-piperidine derivative is optical-active cis-3-amino-2-phenylpiperidine.

## INTERNATIONAL SEARCH REPORT

International application No.
PCT/JP02/13391

A. CLASSIFICATION OF SUBJECT MATTER
Int.Cl$^7$ C07D211/56//C07M7:00

According to International Patent Classification (IPC) or to both national classification and IPC

B. FIELDS SEARCHED

Minimum documentation searched (classification system followed by classification symbols)
Int.Cl$^7$ C07D211/56, C07B57/00

Documentation searched other than minimum documentation to the extent that such documents are included in the fields searched

Electronic data base consulted during the international search (name of data base and, where practicable, search terms used)
REGISTRY(STN), CAPLUS(STN)

C. DOCUMENTS CONSIDERED TO BE RELEVANT

| Category* | Citation of document, with indication, where appropriate, of the relevant passages | Relevant to claim No. |
|---|---|---|
| X | WO 00/18403 A1 (MERCK & CO., INC.), 06 April, 2000 (06.04.00), Example 1 & JP 2002-525325 A & EP 1119356 A1 & US 6241964 B1 & CA 2343106 A | 1-3,9,10 |
| A | WO 94/27966 A1 (PFIZER INC.), 08 December, 1994 (08.12.94), & JP 8-507297 A & EP 700384 A1 & US 6436961 B1 & CA 2162400 A & FI 9505708 A | 1-10 |
| A | WO 01/77100 A2 (PFIZER PRODUCTS INC.), 18 October, 2001 (18.10.01), & EP 1272484 A2 & NO 2002004874 A | 1-3,9 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 March, 2003 (07.03.03) | 18 March, 2003 (18.03.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/13391

C (Continuation). DOCUMENTS CONSIDERED TO BE RELEVANT

| Category* | Citation of document, with indication, where appropriate, of the relevant passages | Relevant to claim No. |
|---|---|---|
| A | EP 1095939 A2 (PFIZER PRODUCTS INC.), 02 May, 2001 (02.05.01), & JP 2001-151773 A & US 6486325 B1 & ZA 2000005695 A & CN 1293193 A & BR 2000004901 A | 1-3,9 |
| A | JP 2001-97933 A (Toray Industries, Inc.), 10 April, 2001 (10.04.01), (Family: none) | 2-8 |
| A | JP 10-218863 A (Toray Industries, Inc.), 18 August, 1998 (18.08.98), (Family: none) | 4-8 |

Form PCT/ISA/210 (continuation of second sheet) (July 1998)